# EUROPEAN PATENT SPECIFICATION

(11) **EP 4 398 256 B1**
(45) Date of publication and mention of the grant of the patent: **28.01.2026**
(21) Application number: 22864243.5
(22) Date of filing: 16.08.2022
(51) Int. Cl.: G16C 20/10, G16C 10/00

(54) **SIMULATION DEVICE, SIMULATION METHOD, AND SIMULATION PROGRAM**
SIMULATIONSVORRICHTUNG, SIMULATIONSVERFAHREN UND SIMULATIONSPROGRAMM
DISPOSITIF DE SIMULATION, PROCÉDÉ DE SIMULATION ET PROGRAMME DE SIMULATION

(30) Priority: 01.09.2021 JP 2021142374
(43) Date of publication of application: 10.07.2024
(73) Proprietor: Resonac Corporation, Tokyo 105-7325 (JP)
(72) Inventor: HIRAKAWA, Teruo, Tokyo 105-8518 (JP); SAKAGUCHI, Suguru, Tokyo 105-8518 (JP); OKUNO, Yoshishige, Tokyo 105-8518 (JP)
(74) Representative: Strehl & Partner mbB
(86) International application number: PCT/JP2022/030968
(87) International publication number: WO 2023/032658

(56) References cited:
- WO-A1-2016/133002
- CN-A- 104 765 918
- JP-A- 2000 107 593
- JP-A- 2005 034 843
- JP-A- 2007 316 976
- JP-A- 2011 221 868
- US-A1- 2018 032 704
- GISSINGER J. R. ET AL: "Modeling chemical reactions in classical molecular dynamics simulations", POLYMER, vol. 128, 18 September 2017 (2017-09-18), pages 211 - 217, XP085214708, ISSN: 0032-3861, DOI: 10.1016/J.POLYMER.2017.09.038
- SALCICCIOLI M. ET AL: "A review of multiscale modeling of metal-catalyzed reactions: Mechanism development for complexity and emergent behavior", CHEMICAL ENGINEERING SCIENCE, OXFORD, GB, vol. 66, no. 19, 30 May 2011 (2011-05-30), pages 4319 - 4355, XP028264661, ISSN: 0009-2509, [retrieved on 20110613], DOI: 10.1016/J.CES.2011.05.050
- MIURA, RYUJI; TSUBOI, HIDEYUKI; KOYAMA, MICHIHISA; ENDO, AKIRA; KUBO, MOMOJI; DEL CARPIO, CARLOS A.; MIYAMOTO, AKIRA: "Development and Application of a Classical Molecular Dynamics Calculation Program Implementing a Function to Express Chemical Reaction Processes", IEICE TECHNICAL REPORT, vol. 105 (SDM2005-185), no. 318, 30 September 2005 (2005-09-30), pages 27 - 29, XP009544287

## Description

### TECHNICAL FIELD

The present disclosure relates to simulation devices, simulation methods, and simulation programs.

### BACKGROUND ART

A simulation device that analyzes a reaction mechanism by molecular dynamics computation is known. According to this simulation device, the molecular dynamics computation and identification of a reaction product are performed for each predetermined time step after molecular information, reaction conditions, or the like are set, thus enabling a reaction mechanism to be analyzed.

### PRIOR ART DOCUMENTS

### PATENT DOCUMENTS

Patent Document 1: Japanese Laid-Open Patent Publication No. 2005-34843
Patent Document 2: International Publication Pamphlet No. WO 2016/133002

### DISCLOSURE OF THE INVENTION

### PROBLEM TO BE SOLVED BY THE PRESENT INVENTION

However, in the case of the simulation device described above, in order to promote the reaction within a realistic analysis time, it is necessary to set a high temperature that is far from reality as the reaction condition. For this reason, a reaction that cannot occur or hardly occurs at low temperatures (that is, in reality) is also output as a simulation result, and it is difficult to perform a useful analysis on the reaction mechanism.

The present disclosure enables a more useful analysis to be performed when analyzing the reaction mechanism by the molecular dynamics computation.

### MEANS OF SOLVING THE PROBLEM

A simulation device according to a first aspect of the present disclosure includes a determination unit configured to compute a distance between an atom forming a first molecule and an atom forming a second molecule while performing a molecular dynamics computation, and determine whether or not the first molecule and the second molecule are in close proximity of each other; an imparting unit configured to impart a parameter for increasing a reaction probability with respect to the first molecule and/or the second molecule in a case where the determination unit determines that the first molecule and the second molecule are in close proximity of each other; an elementary reaction generation unit configured to generate an elementary reaction in a transition state, based on a chemical reaction identified in the molecular dynamics computation; and a reaction rate constant computation unit configured to compute a reaction rate constant of the generated elementary reaction.

According to a second aspect of the present disclosure, the simulation device according to the first aspect of the present disclosure further includes a setting unit configured to set a time interval at which the imparting unit imparts the parameter before performing the molecular dynamics computation, wherein the imparting unit imparts the parameter in a case where the determination unit determines the close proximity and the time interval elapses from a previous imparting.

According to a third aspect of the present disclosure, the setting unit of the simulation device according to the second aspect of the present disclosure sets a time interval that does not affect a temperature of an entire reaction system, and is required for a molecule after the chemical reaction to stabilize.

According to a fourth aspect of the present disclosure, the simulation device according to the first aspect of the present disclosure further includes a setting unit configured to set a target to which the parameter is not to be imparted by the imparting unit before performing the molecular dynamics computation, wherein the imparting unit does not impart the parameter with respect to each of the first molecule and the second molecule even in a case where the determination unit determines the proximity when both of the first molecule and the second molecule are targets to which the parameter is not to be imparted.

According to a fifth aspect of the present disclosure, in the simulation device according to the fourth aspect of the present disclosure, the setting unit sets a target to which the parameter is not to be imparted by the imparting unit, by specifying a type of atom or an index of atom, and the imparting unit does not impart the parameter with respect to each of the first molecule and the second molecule even in a case where the determination unit determines that the close proximity when both of the first molecule and the second molecule are molecules including the specified atom.

According to a sixth aspect of the present disclosure, the simulation device according to the first aspect of the present disclosure further includes a position computation unit configured to identify atom-to-atom existing within a range of a bond distance as the first molecule or the second molecule.

According to a seventh aspect of the present disclosure, the determination unit of the simulation device according to the sixth aspect of the present disclosure determines that the first molecule and the second molecule are in close proximity of each other in a case where the distance between the atom included in the first molecule and the atom included in the second molecule is a predetermined multiple or less of the bond distance.

According to an eighth aspect of the present disclosure, the simulation device according to the seventh aspect of the present disclosure further includes a setting unit configured to set the parameter to be imparted by the imparting unit before performing the molecular dynamics computation, wherein the imparting unit imparts information related to a temperature, based on the parameter set by the setting unit.

According to a ninth aspect of the present disclosure, imparting unit of the simulation device according to the eighth aspect of the present disclosure imparts a kinetic energy in a colliding direction, based on the parameter set by the setting unit.

According to a tenth aspect of the present disclosure, the setting unit of the simulation device according to an eighth aspect of the present disclosure sets the information related to the temperature to be imparted as the parameter.

According to an eleventh aspect of the present disclosure, the setting unit of the simulation device according to the ninth aspect of the present disclosure sets the kinetic energy to be imparted as the parameter.

According to a twelfth aspect of the present disclosure, the setting unit of the simulation device according to a ninth aspect of the present disclosure sets a translational velocity to be imparted as the parameter.

A simulation method according to a thirteenth aspect of the present disclosure includes the steps of determining whether or not a first molecule and a second molecule are in close proximity of each other, by computing a distance between an atom forming the first molecule and an atom forming the second molecule while performing a molecular dynamics computation; imparting a parameter for increasing a reaction probability with respect to the first molecule and/or the second molecule in a case where the determining step determines that the first molecule and the second molecule are in close proximity of each other; generating an elementary reaction in a transition state, based on a chemical reaction identified in the molecular dynamics computation; and computing a reaction rate constant of the generated elementary reaction.

A simulation program according to a fourteenth aspect of the present disclosure causes a computer to perform the steps of determining whether or not a first molecule and a second molecule are in close proximity of each other, by computing a distance between an atom forming the first molecule and an atom forming the second molecule while performing a molecular dynamics computation; imparting a parameter for increasing a reaction probability with respect to the first molecule and/or the second molecule in a case where the determining step determines that the first molecule and the second molecule are in close proximity of each other; generating an elementary reaction in a transition state, based on a chemical reaction identified in the molecular dynamics computation; and computing a reaction rate constant of the generated elementary reaction.

### EFFECTS OF THE PRESENT INVENTION

According to the present disclosure, a more useful analysis can be performed when analyzing the reaction mechanism by the molecular dynamics computation.

### BRIEF DESCRIPTION OF THE DRAWINGS

[FIG. 1] FIG. 1 is a diagram for explaining an overview of a simulation device.
[FIG. 2] FIG. 2 is a diagram illustrating an example of a hardware configuration of the simulation device.
[FIG. 3] FIG. 3 is a diagram illustrating an example of a functional configuration of the simulation device.
[FIG. 4] FIG. 4 is a flow chart illustrating procedures of a simulation process.
[FIG. 5] FIG. 5 is a first flow chart illustrating procedures of a molecular dynamics computation process.
[FIG. 6] FIG. 6 is a second flow chart illustrating procedures of the molecular dynamics computation process.
[FIG. 7] FIG. 7 is a flow chart illustrating procedures of a close proximity molecule determination process.
[FIG. 8] FIG. 8 is a diagram illustrating a specific example of the close proximity molecule determination process.
[FIG. 9] FIG. 9 is a flow chart illustrating procedures of a reaction probability varying process.
[FIG. 10] FIG. 10 is a diagram illustrating a specific example of the reaction probability varying process.
[FIG. 11] FIG. 11 is a diagram illustrating examples of processed results of the molecular dynamics computation process.
[FIG. 12] FIG. 12 is a diagram illustrating examples of elementary reaction data.

### MODE OF CARRYING OUT THE INVENTION

Hereinafter, each of embodiments will be described with reference to the accompanying drawings. In the present specification and drawings, constituent elements having substantially the same functional configuration are designated by the same reference numerals, and a redundant description thereof will be omitted.

### [First Embodiment]

### <Overview of Simulation Device>

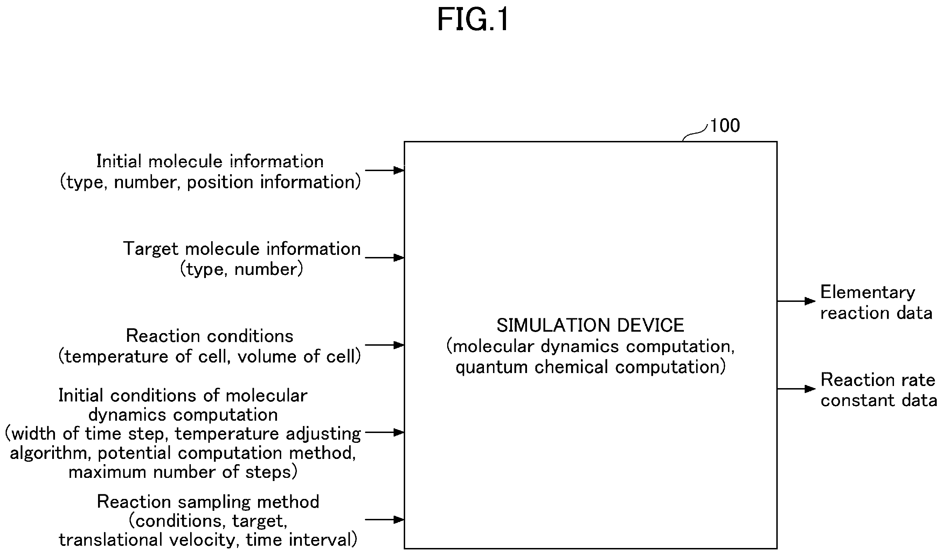

First, an overview of a simulation device according to a first embodiment will be described. FIG. 1 is a diagram for explaining the overview of the simulation device. In the present embodiment, the simulation device 100 generates an elementary reaction by analyzing a reaction mechanism by molecular dynamics computation, and computes a reaction rate constant by quantum chemical computation.

The molecular dynamics computation refers to a process of simulating physical movements of atoms and molecules by Molecular Dynamics (MD). In the present embodiment, the reaction mechanism is analyzed by simulating the physical movements of atoms and molecules by molecular dynamics computation, to generate the elementary reaction. The reaction refers to a phenomenon in which the atoms forming the molecule form a new molecule, and includes generation of a new bond or elimination of the bond.

In addition, quantum chemical computation refers to a process of analyzing structures and properties of atoms and molecules from electronic states thereof. In the present embodiment, the reaction rate constant is computed from the analysis results of the reaction mechanism, by performing the quantum chemical computation.

As illustrated in FIG. 1, when operating the simulation device 100, a user inputs, as initial molecule information, a type of initial molecule, a number of each initial molecule, position information of each initial molecule within a cell, or the like.

In addition, when operating the simulation device 100, the user inputs, as target molecule information, a type of target molecule and a number of each target molecule. The "target molecule" is a molecule that is not included in the initial molecule information, and refers to a molecule (product) that is expected to be finally generated by performing the molecular dynamics computation.

Moreover, when operating the simulation device 100, the user inputs, as reaction conditions, an initial temperature of the cell for which molecular dynamics computation is performed, a cell volume, or the like. The temperature that is input in this case is assumed to be a temperature (a temperature (for example, a low temperature of 300K, 450K, or the like) at which a realistic reaction is likely to occur) at which the molecules are moderately mixed due to thermal fluctuation. The initial temperature that is input is controlled to be substantially constant by a heat bath (an algorithm for adjusting the temperature) while performing the molecular dynamics computation.

Further, when operating the simulation device 100, the user inputs initial conditions of the molecular dynamics computation. The initial conditions of the molecular dynamics computation are conditions required to perform the molecular dynamics computation, and refer to various conditions other than the conditions input as the reaction conditions. More particularly, the initial conditions of the molecular dynamics computation include, among other things:
- A width (Δt) of time step when performing the molecular dynamics computation;
- ON/OFF setting of the algorithm for adjusting the temperature;
- Selection of the computation method to compute a force (potential) acting between the atoms; and
- A maximum number of steps.

The width (Δt) of the time step refers to a time interval of every one operation of artificially imparting kinetic energy to each molecule when performing the molecular dynamics computation.

In addition, when operating the simulation device 100, the user inputs a reaction sampling method. In the present embodiment, the reaction sampling method includes, among other things:
- Conditions for imparting a translational velocity;
- A target to which the translational velocity is not to be imparted;
- The translational velocity to be imparted; and
- A time interval at which the translational velocity is to be imparted.

The translational velocity is an example of a "parameter for increasing a reaction probability", and indicates a velocity at which the molecule within the cell undergoes parallel translation in a reaction system. In a case where the movement of the molecule is a movement only at the translational velocity, a relative positional relationship of the atoms in the molecule does not vary. Hence, by imparting the translational velocity to the molecule, the reaction probability of the molecule can be increased without varying the relative positional relationship of the atoms in the molecule.

As illustrated in FIG. 1, the simulation device 100 operates under the initial molecule information, the target molecule information, the reaction conditions, the initial conditions of molecular dynamics computation, and the reaction sampling method that are input, and analyzes the reaction mechanism by the molecular dynamics computation to generate the elementary reaction. Further, the simulation device 100 analyzes the structures and properties of the atoms and the molecules from the electronic state by the quantum chemical computation, and computes the reaction rate constant from the analysis results of the reaction mechanism.

As described above, the simulation device 100 according to the first embodiment includes:
- Before performing the molecular dynamics computation, inputting a temperature (for example, a low temperature of 300K, 450K, or the like) at which a realistic reaction is likely to occur, as the reaction condition; and
- While performing the molecular dynamics computation, imparting a parameter for increasing the reaction probability according to the reaction sampling method.

In the related art, because the initial temperature of the cell is set to a high temperature as the reaction condition, even a reaction which cannot occur or hardly occurs at low temperatures are output as the simulation result. In contrast, according to the present embodiment, such reactions are less likely to be output as the simulation results.

In addition, in the related art, the reaction is not promoted in a case where a low temperature is set as the reaction condition. In contrast, according to the present embodiment, because the parameter for increasing the reaction probability is imparted while performing the molecular dynamics computation, the reaction can be promoted even when a low temperature is set as the reaction condition.

As a result, according to the present embodiment, a reaction that can actually occur is more likely to be output, and it is possible to perform a more useful analysis when analyzing the reaction mechanism by the molecular dynamics computation.

### <Hardware Configuration of Simulation Device>

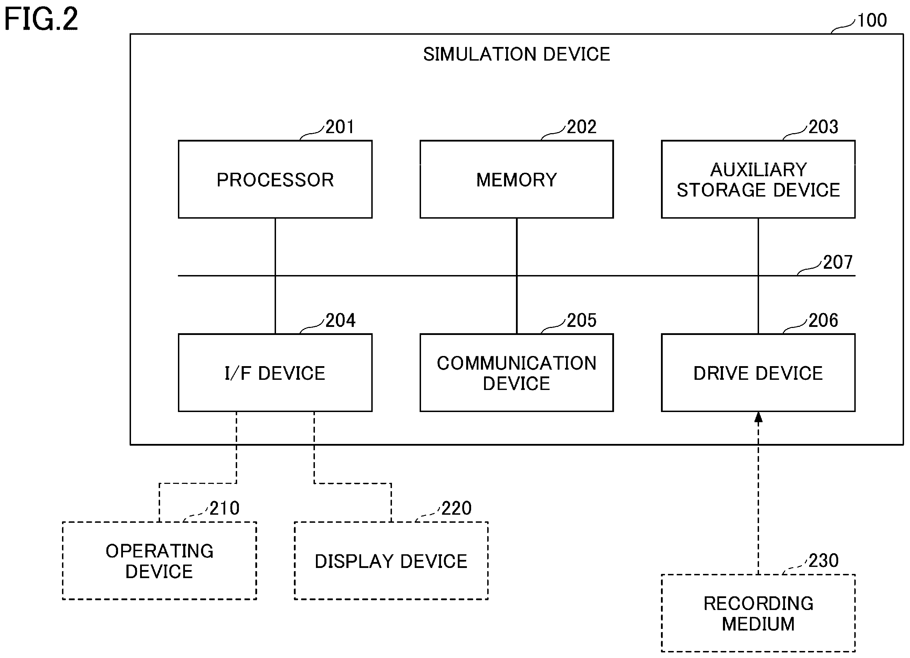

Next, a hardware configuration of the simulation device 100 will be described. FIG. 2 is a diagram illustrating an example of the hardware configuration of the simulation device. As illustrated in FIG. 2, the simulation device 100 includes a processor 201, a memory 202, an auxiliary storage device 203, an interface (I/F) device 204, a communication device 205, and a drive device 206. Hardware components of the simulation device 100 are connected to one another via a bus 207.

The processor 201 includes various computing devices, such as a central processing unit (CPU), a graphics processing unit (GPU), or the like. The processor 201 reads various programs (for example, a simulation program which will be described later, or the like) into the memory 202, and executes the programs.

The memory 202 includes a main storage device, such as a read only memory (ROM), a random access memory (RAM), or the like. The processor 201 and the memory 202 form a so-called computer, and the computer performs various functions by executing various programs read into the memory 202 by the processor 201.

The auxiliary storage device 203 stores various programs, and various data that are used when the processor 201 executes the various programs.

The I/F device 204 is a connection device that connects to an operating device 210 and a display device 220, which are examples of external devices. The communication device 205 is a communication device for communicating with an external device that is not illustrated, via a network.

The drive device 206 is a device on which a recording medium 230 is set. The recording medium 230 includes a medium for optically, electrically, or magnetically recording information, such as a CD-ROM, a flexible disk, a magneto-optical disk, or the like. The recording medium 230 may include a semiconductor memory or the like that electrically records information, such as a ROM, a flash memory, or the like.

The various programs installed in the auxiliary storage device 203 are installed by setting a distributed recording medium 230 in the drive device 206 and reading the various programs recorded in the recording medium 230 by the drive device 206, for example. Alternatively, the various programs installed in the auxiliary storage device 203 may be installed by being downloaded from the network via the communication device 205.

### <Functional Configuration of Simulation Device>

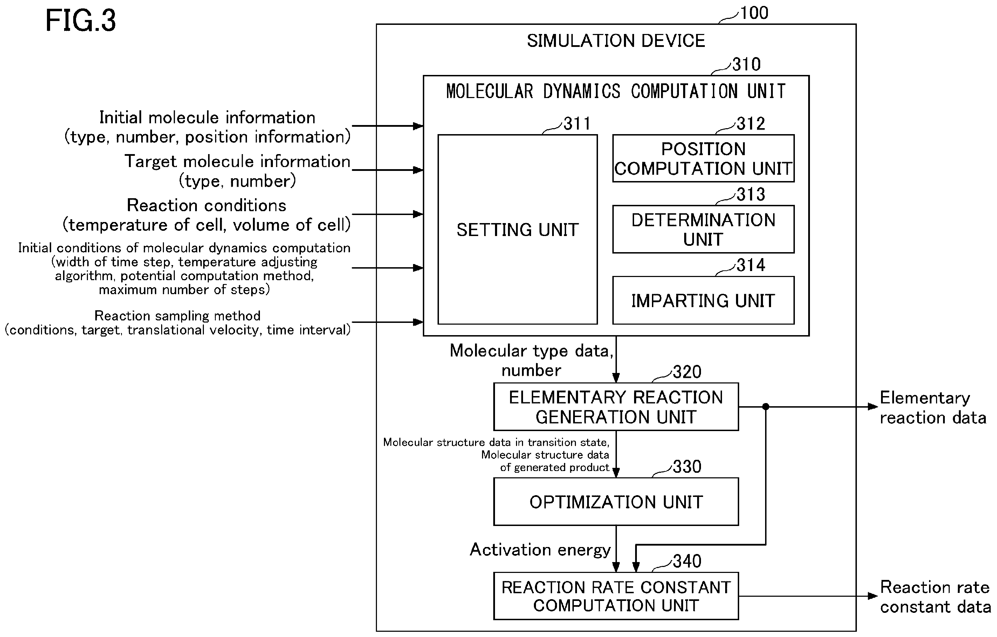

Next, a functional configuration of the simulation device 100 will be described. FIG. 3 is a diagram illustrating an example of the functional configuration of the simulation device. As described above, the simulation program is installed in the simulation device 100, and the simulation device 100 executes the simulation program to function as:
- A molecular dynamics computation unit 310;
- An elementary reaction generation unit 320;
- An optimization unit 330; and
- A reaction rate constant computation unit 340.

The molecular dynamics computation unit 310 performs the molecular dynamics computation based on the initial molecule information, the target molecule information, the reaction conditions, the initial conditions of the molecular dynamics computation, and the reaction sampling method input by the user.

More particularly, as illustrated in FIG. 3, the molecular dynamics computation unit 310 includes a setting unit 311, a position computation unit 312, a determination unit 313, and an imparting unit 314.

Before performing the molecular dynamics computation, the setting unit 311 receives the input of the initial molecule information, the target molecule information, the reaction conditions, the initial conditions of the molecular dynamics computation, and the reaction sampling method. In addition, the setting unit 311 sets the initial molecule information, the target molecule information, the reaction conditions, and the initial conditions of the molecular dynamics computation that are received, in the position computation unit 312. Moreover, the setting unit 311 sets the reaction sampling method that is received, in the determination unit 313 and the imparting unit 314.

The position computation unit 312 imparts kinetic energy to each molecule within the cell, for every width (Δt) of the time step, and computes position information of a movement destination, from a translational velocity based on the imparted kinetic energy.

The position computation unit 312 determines whether or not a molecule-to-molecule chemical reaction is generated, based on the computed position information. In a case where the position computation unit 312 determines that the chemical reaction is generated, the position computation unit 312 identifies a molecule generated by the chemical reaction, and counts up a number of molecular type data indicating the type of the identified molecule. Further, the position computation unit 312 notifies the elementary reaction generation unit 320 of the molecular type data and the counted up number of molecular type data.

In addition, the position computation unit 312 identifies a molecule based on the position information of each atom within the cell, for every width (Δt) of the time step, and notifies the determination unit 313 of the identified molecule together with the position information of the atom included in each molecule.

When the imparting unit 314 imparts the kinetic energy to each molecule within the cell, the determination unit 313 determines whether or not each molecule is a close proximity molecule, based on the position information of the atom included in each molecule.

More particularly, the determination unit 313 determines whether or not the position information of the atom included in each molecule satisfies the "conditions for imparting the translational velocity" included in the reaction sampling method, that is, whether or not a first molecule and a second molecule are in close proximity of each other by computing a distance between the atoms included in the first molecule and the atom included in the second molecule, and the determination unit 106 determines that the first molecule and the second molecule are in close proximity of each other in a case where atoms existing at a predetermined number multiple of a bond distance or less (for example, 1.5 times or less) are identified.

The determination unit 313 notifies the imparting unit 314 of the molecules determined to be the close proximity molecules, for every width (Δt) of the time step.

The imparting unit 314 imparts the "translational velocity to be imparted" included in the reaction sampling method, with respect to the first molecule and the second molecule that are determined to be in close proximity of each other by the determination unit 313. Thus, the position computation unit 312 can compute the position information of the movement destination of each molecule within the cell in a state where the translational velocity is imparted by the imparting unit 314, and determine whether or not a molecule-to-molecule chemical reaction is generated based on the computed position information.

Accordingly, in the present embodiment, the imparting unit 314 imparts, as the parameter for increasing the reaction probability, "the translational velocity" including:
- A function of promoting an intermolecular reaction by causing a molecule-to-molecule collision; and
- A function of indirectly promoting the intermolecular reaction.

The translational velocity of each molecule before being imparted by the imparting unit 314 can be computed from the kinetic energy imparted to each molecule by the position computation unit 312, for example, based on a gross mass number of the molecule. Accordingly, the reaction is promoted as the kinetic energy imparted to each molecule by the position computation unit 312 increases. In addition, the reaction is further promoted (that is, the reaction probability is further increased) when the translational velocity is imparted by the imparting unit 314. However, if the translational velocity imparted by the imparting unit 314 is too high, a temperature of the entire reaction system may become affected. Hence, when inputting the "translational velocity to be imparted" as the reaction sampling method, the user desirably inputs the translational velocity within a range to such an extent that does not affect the temperature of the entire reaction system.

In addition, the imparting unit 314 imparts the "translational velocity to be imparted" included in the reaction sampling method, with respect to the molecule other than the "target to which the translational velocity is not to be imparted" included in the reaction sampling method, among the molecules determined by the determination unit 313 as the molecules to which the parameter for increasing the reaction probability is to be imparted.

Hence, even in the case of a molecule to which the parameter that increases the reaction probability is imparted, by configuring the molecule so as not to promote the reaction with respect to the "target to which the translational velocity is not to be imparted", it is possible to avoid promoting a reaction that is not useful to the user, and reduce a computation time of the molecular dynamics computation, for example.

Moreover, when imparting the translational velocity, the imparting unit 314 refers to the "time interval for imparting the translational velocity" included in the reaction sampling method, and determines whether or not the referred time interval has elapsed from the previous imparting of the translational velocity. Then, in a case where the imparting unit 314 determines that the time interval referred to has not elapsed from the previous imparting of the translational velocity, the imparting unit 314 does not impart the translational velocity, and in a case where the imparting unit 314 determines that the time interval referred to has elapsed from the previous imparting of the translational velocity, the imparting unit 314 imparts the translational velocity.

Accordingly, by adopting a configuration in which the translational velocity is imparted at the time interval, it is possible to avoid the temperature of the entire reaction system from becoming high, and by imparting the translational velocity, it is possible to avoid the generation of a reaction that cannot actually occur or a reaction that hardly occurs. In other words, when inputting the "time interval for imparting the translational velocity" as the reaction sampling method, the user desirably inputs a time interval that does not affect the temperature of the entire reaction system and is required for the molecule to stabilize after the chemical reaction.

The elementary reaction generation unit 320 generates an elementary reaction in a transition state, based on the chemical reaction identified in the molecular dynamics computation by the molecular dynamics computation unit 310, outputs elementary reaction data, and outputs molecular structure data in the transition state.

In addition, the elementary reaction generation unit 320 outputs the molecular structure data of a product generated at a point in time when the molecular dynamics computation by the molecular dynamics computation unit 310 ends.

The optimization unit 330 computes an activation energy by quantum chemical computation, so as to optimize the molecular structural data in the transition state and the molecular structural data of the product computed by the elementary reaction generation unit 320. Further, the optimization unit 330 notifies the reaction rate constant computation unit 340 of the computed activation energy.

The reaction rate constant computation unit 340 computes a reaction rate constant from a reaction rate equation, based on the elementary reaction data and the activation energy, and outputs the reaction rate constant as reaction rate constant data of the identified elementary reaction.

The reaction rate constant computation unit 340 outputs the computed reaction rate constant to the display device 220, for example, as the reaction rate constant data of the identified elementary reaction.

### <Procedures of Simulation Process>

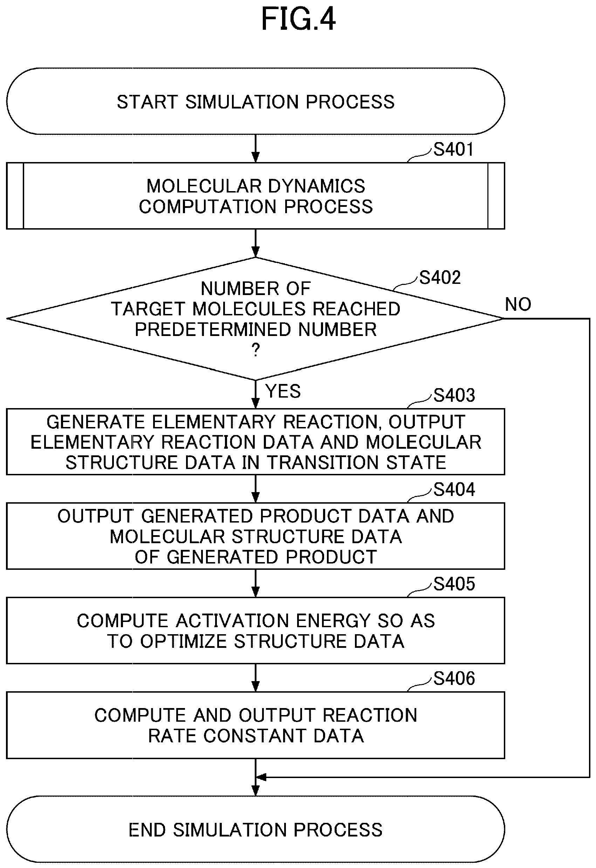

Next, procedures of a simulation process performed by the simulation device 100 will be described. FIG. 4 is a flow chart illustrating the procedures of the simulation process.

In step S401, the molecular dynamics computation unit 310 performs a molecular dynamics computation process to analyze the reaction mechanism. Details of the molecular dynamics computation process will be described later.

In step S402, the elementary reaction generation unit 320 branches depending on whether the number of target molecules reached a predetermined number, or the number of target molecules did not reach the predetermined number and the number of steps of the molecular dynamics computation reached a predetermined maximum number of steps, in the molecular dynamics computation process, and ends the simulation process in a case where the number of target molecules did not reach the predetermined number and the number of steps of the molecular dynamics computation reached the predetermined maximum number of steps (in a case where decision in step S402 is NO).

On the other hand, in step S402, in a case where the number of target molecules reached the predetermined number in the molecular dynamics computation process (in a case where decision in step S402 is YES), the process proceeds to step S403.

In step S403, the elementary reaction generation unit 320 generates the elementary reaction in the transition state, based on the chemical reaction generated in each time step of the molecular dynamics computation process, and outputs the molecular structure data together with the elementary reaction data.

In step S404, the elementary reaction generation unit 320 outputs the molecular structure data of a product together with generated product data, for the product generated at a point in time when the number of target molecules reaches the predetermined number in the molecular dynamics computation process.

In step S405, the optimization unit 330 computes the activation energy, so as to optimize the molecular structure data in the transition state output in step S403 and the molecular structure data of the product output in step S404.

In step S406, the reaction rate constant computation unit 340 computes the reaction rate constant, based on the elementary reaction data output in step S403 and the activation energy computed in step S405, and outputs the reaction rate constant data including the reaction rate constant.

### <Procedures of Molecular Dynamics Computation Process>

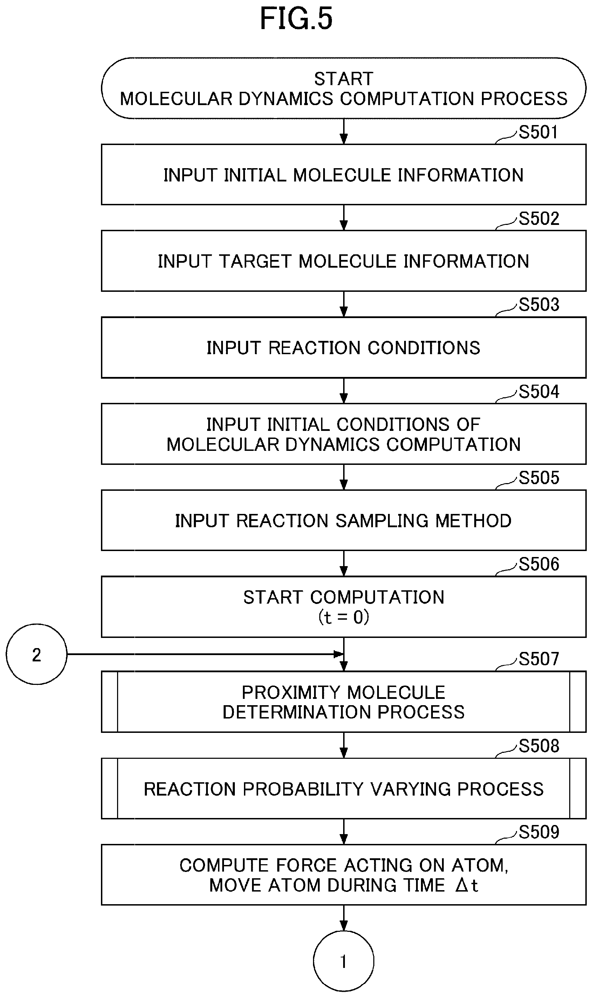

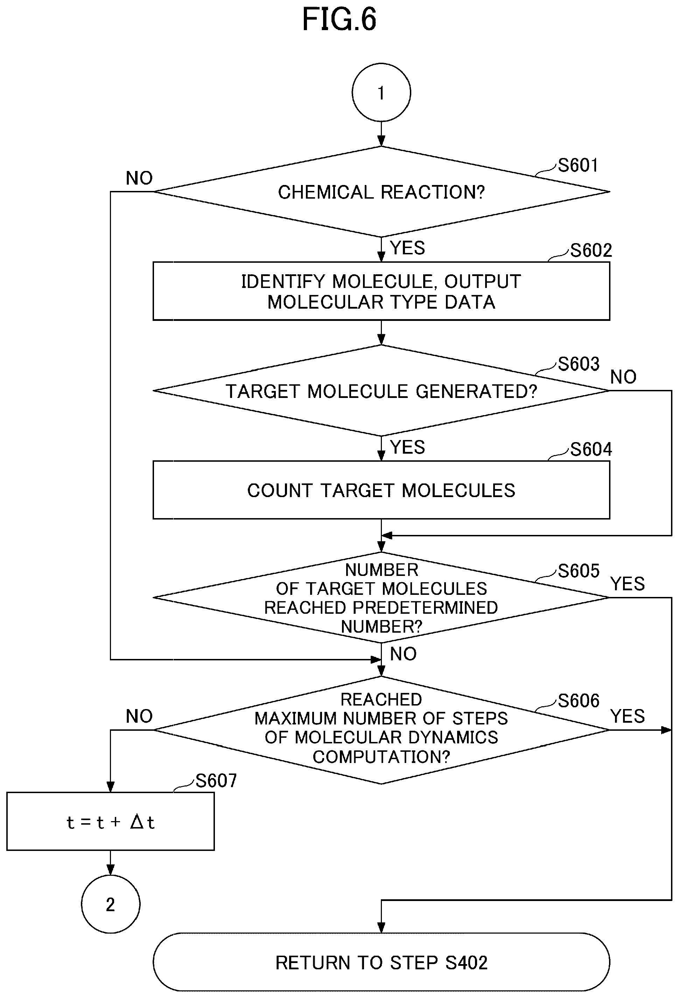

Next, procedures of the molecular dynamics computation process (step S401 in FIG. 4) by the molecular dynamics computation unit 310 will be described. FIG. 5 and FIG. 6 are first and second flow charts illustrating the procedures of the molecular dynamics computation process, respectively.

In step S501, the molecular dynamics computation unit 310 receives the input of the initial molecular information.

In step S502, the molecular dynamics computation unit 310 receives the input of the target molecular information.

In step S503, the molecular dynamics computation unit 310 receives the input of the reaction conditions.

In step S504, the molecular dynamics computation unit 310 receives the input of the initial conditions of the molecular dynamics computation.

In step S505, the molecular dynamics computation unit 310 receives the input of the reaction sampling method.

In step S506, the molecular dynamics computation unit 310 starts the molecular dynamics computation in response to a start instruction from the user instructing to start the molecular dynamics computation. In the molecular dynamics computation unit 310, a time step t at the start is set to zero.

In step S507, the molecular dynamics computation unit 310 performs a close proximity molecule determination process. The close proximity molecule determination process is a process of determining a molecule to which the parameter for increasing the reaction probability is imparted within the cell, and details thereof will be described later. The molecular dynamics computation unit 310 determines that the molecule is the molecule to which the parameter for increasing the reaction probability is imparted when the "conditions for imparting the translational velocity" included in the reaction sampling method are satisfied.

In step S508, the molecular dynamics computation unit 310 performs a reaction probability varying process. The reaction probability varying process is a process of imparting the translational velocity, as the parameter for increasing the reaction probability, to the molecule that is determined as the molecule to which the parameter for increasing the reaction probability is to be imparted, and details thereof will be described later.

In step S509, the molecular dynamics computation unit 310 computes a force acting on the atom by imparting kinetic energy to each molecule within the cell, and computes position information of the movement destination of each molecule after the width (Δt) of the time step elapses. In this state, in a case where the translational velocity is imparted in step S508, the molecular dynamics computation unit 310 computes the position information of the movement destination in a state where the translational velocity is imparted.

Next, in step S601 illustrated in FIG. 6, the molecular dynamics computation unit 310 determines whether or not a chemical reaction is generated. In a case where step S601 determines that no chemical reaction is generated (in a case where decision in step S601 is NO), the process proceeds to step S606.

On the other hand, in a case where step S601 determines that a chemical reaction is generated (in a case where decision in step S601 is YES), the process proceeds to step S602.

In step S602, the molecular dynamics computation unit 310 identifies the molecule generated by the chemical reaction, and outputs molecular type information indicating the type of the identified molecule.

In step S603, the molecular dynamics computation unit 310 determines whether or not the target molecule is generated. In a case where step S603 determines that the target molecule is not generated (in a case where decision in step S603 is NO), the process proceeds to step S605.

On the other hand, in a case where step S603 determines that the target molecule is generated (in a case where decision in step S603 is YES), the process proceeds to step S604.

In step S604, the molecular dynamics computation unit 310 counts up the number of target molecules.

In step S605, the molecular dynamics computation unit 310 determines whether or not the number of target molecules reached a predetermined number. In a case where step S605 determines that the number of target molecules has not reached the predetermined number (in a case where decision in step S605 is NO), the process proceeds to step S606.

In step S606, the molecular dynamics computation unit 310 determines whether or not the number of steps of the molecular dynamics computation reached a predetermined maximum number of steps. In a case where step S606 determines that the maximum number of steps was not reached (in a case where decision in step S606 is NO), the process proceeds to step S607.

In step S607, the molecular dynamics computation unit 310 advances the time step by Δt. Thereafter, the process returns to step S507 illustrated in FIG. 5.

On the other hand, in a case where step S606 determines that the number of steps of the molecular dynamics computation reached the maximum number of steps (in a case where decision in step S606 is YES), the molecular dynamics computation process ends, and the process returns to step S402 illustrated in FIG. 4. In this case, step S402 determines that the number of target molecules did not reach the predetermined number (decision in step S402 is NO), and the simulation process ends.

In a case where step S605 determines that the number of target molecules reached the predetermined number (in a case where decision in step S605 is YES), the molecular dynamics computation process ends, and the process returns to step S402 illustrated in FIG. 4. In this case, step S402 determines that the number of target molecules reached the predetermined number (decision in step S402 is YES), and the process proceeds to step S403.

The processes of steps S403 through S406 in the present embodiment is described as being performed only when the number of target molecules reaches the predetermined number in step S605, but the processes of steps S403 through S406 may be performed every time the target molecule is generated, the elementary reaction in the transition state may be successively generated based on the identified chemical reaction, and the reaction rate constant of the generated elementary reaction may be computed. That is, after the process of step S604 is performed, the process may return to the process of step S403, the elementary reaction in the transition state may be generated based on the chemical reaction identified, and the reaction rate constant of the generated elementary reaction may be computed, according to steps S403 through S406. In this case, the process may return to the process of step S605 after the process of step S406 until the number of target molecules reaches the predetermined number.

### <Details of Each Step of Molecular Dynamics Computation Process>

Next, the molecular dynamics computation process (FIG. 5 and FIG. 6) will be described in detail with respect to the close proximity molecule determination process (step S507) and the reaction probability varying process (step S508).

### (1) Details of Close Proximity Molecule Determination Process

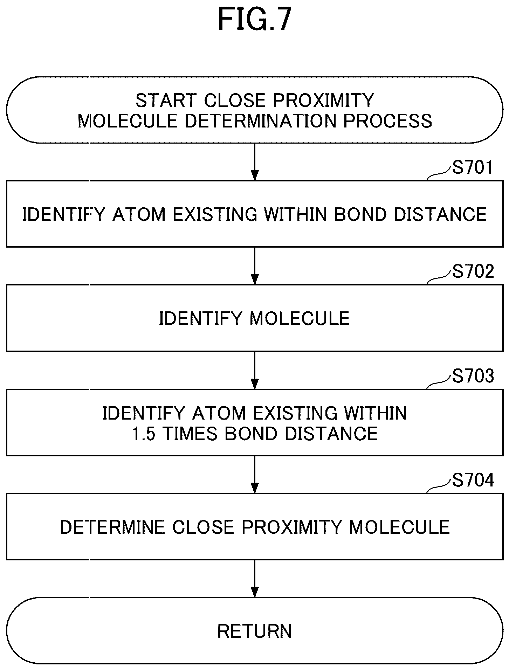

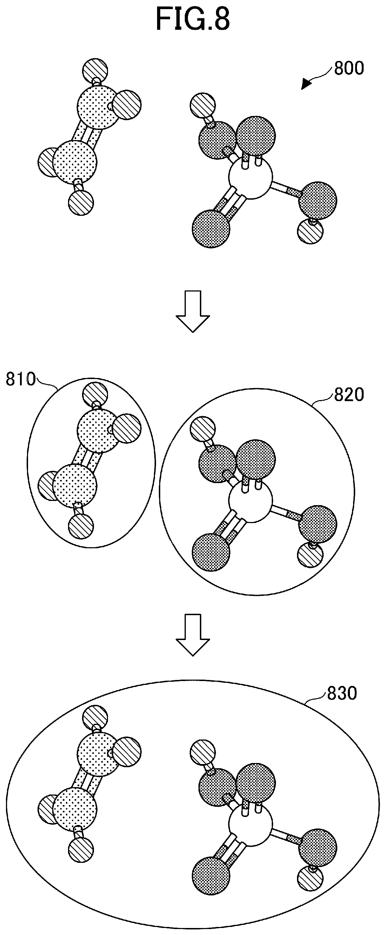

First, the details of the close proximity molecule determination process (step S507) will be described with reference to FIG. 7 and FIG. 8. FIG. 7 is a flow chart illustrating procedures of the close proximity molecule determination process. FIG. 8 is a diagram illustrating a specific example of the close proximity molecule determination process. The flow chart illustrated in FIG. 7 will be described below with reference to FIG. 8.

In step S701, the position computation unit 312 identifies atoms existing within a predetermined bond distance, with respect to all of the atoms within the cell.

In step S702, the position computation unit 312 identifies molecules within the cell. In FIG. 8, a reference numeral 800 denotes an example of the atoms distributed within the cell. In FIG. 8, reference numerals 810 and 820 indicate states where the atoms are identified as existing within the predetermined bond distance in the cell, respectively. More particularly, a group of atoms identified by the reference numeral 810 indicate the atom-to-atom identified as one molecule, and a group of atoms identified by the reference numeral 820 indicate the atom-to-atom identified as one molecule different from the one molecule identified by the reference numeral 810. The bond distance can be determined based on the van der Waals' radius of each atomic species, for example, and can be represented by a sum of the van der Waals' radii of two atoms.

In step S703, the determination unit 313 identifies atoms existing within a predetermined number multiple or less (for example, 1.5 times or less) of the bond distance.

In step S704, the determination unit 313 determines that the molecules to which the atom-to-atom identified in step S703 belong are the close proximity molecules. In FIG. 8, a reference numeral 830 indicates a state where atoms present within a predetermined number multiple or less (for example, 1.5 times or less) of the bond distance are identified, and a molecule (first molecule) indicated by the reference numeral 810 and a molecule (second molecule) indicated by the reference numeral 820 are determined as the close proximity molecules.

### (2) Details of Reaction Probability Varying Process

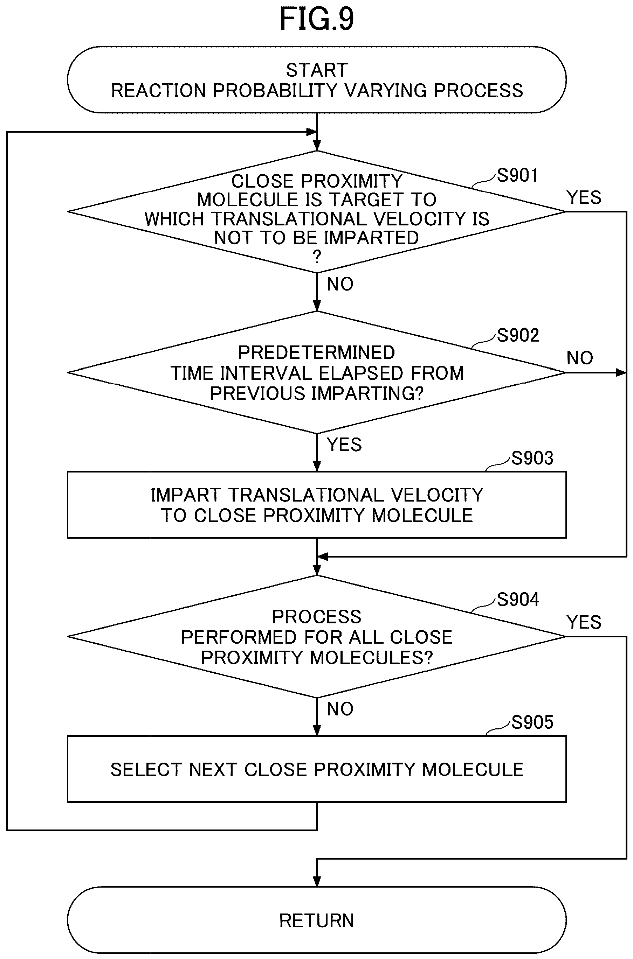

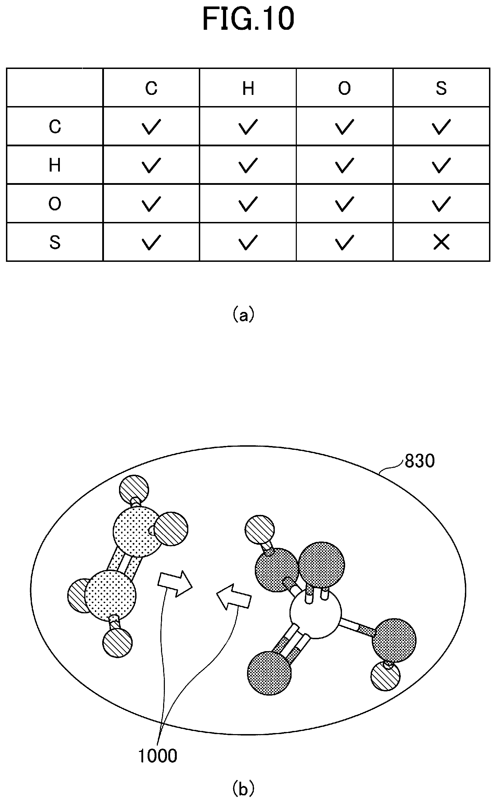

Next, the details of the reaction probability varying process (step S508) will be described with reference to FIG. 9 and FIG. 10. FIG. 9 is a flow chart illustrating procedures of the reaction probability varying process. FIG. 10 is a diagram illustrating a specific example of the reaction probability varying process. The flow chart illustrated in FIG. 9 will be described below with reference to FIG. 10.

In step S901, the imparting unit 314 determines whether or not the molecules identified as the close proximity molecules are the "target to which the translational velocity is not to be imparted". FIG. 10 (a) illustrates an example of a table to be referred to when determining whether or not the molecules are the "target to which the translational velocity is not to be imparted". In FIG. 10 (a), "C", "H"", "O", and "S" all represent atomic symbols, and the example illustrated in FIG. 10 (a) indicates that in a case where the close proximity molecules are molecules including "S", respectively, the close proximity molecules are determined to be the "target to which the translational velocity is not to be imparted".

In a case where step S901 determines that the close proximity molecules are the "target to which the translational velocity is not to be imparted" (in a case where decision in step S901 is YES), the process proceeds to step S904.

On the other hand, in a case where step S901 determines that the close proximity molecules are not the "target to which the translational velocity is not to be imparted" (in a case where decision in step S901 is NO), the process proceeds to step S902.

In step S902, the imparting unit 314 determines whether or not the "time interval for imparting the translational velocity" elapsed from the previous imparting of the translational velocity. In a case where step S902 determines that the "time interval for imparting the translational velocity" has not elapsed (in a case where decision in step S902 is NO), the process proceeds to step S904.

On the other hand, in a case where step S902 determines that the "time interval for imparting the translational velocity" elapsed (in a case where decision in step S902 is YES), the process proceeds to step S903.

In step S903, the imparting unit 314 imparts the translational velocity in a direction in which the close proximity molecules collide with each other. In the close proximity molecules denoted by the reference numeral 830 in FIG. 10 (b), arrows 1000 indicate a state where the translational velocity is imparted in the colliding direction.

In step S904, the imparting unit 314 determines whether or not the processes of steps S901 through S904 were performed for all of the close proximity molecules determined in step S704 illustrated in FIG. 7.

In a case where step S904 determines that there are close proximity molecules for which the processes were not performed (in a case where decision in step S904 is NO), the process proceeds to step S905.

In step S905, the molecular dynamics computation unit 310 selects the next close proximity molecules for which the processes were not performed, and the process returns to step S901.

On the other hand, in a case where step S904 determines that the processes were performed for all of the close proximity molecules (in a case where decision in step S904 is YES), the process returns to step S509 in FIG. 5.

### <Specific Examples of Processed Results of Molecular Dynamics Calculation Process and Elementary Reaction Data>

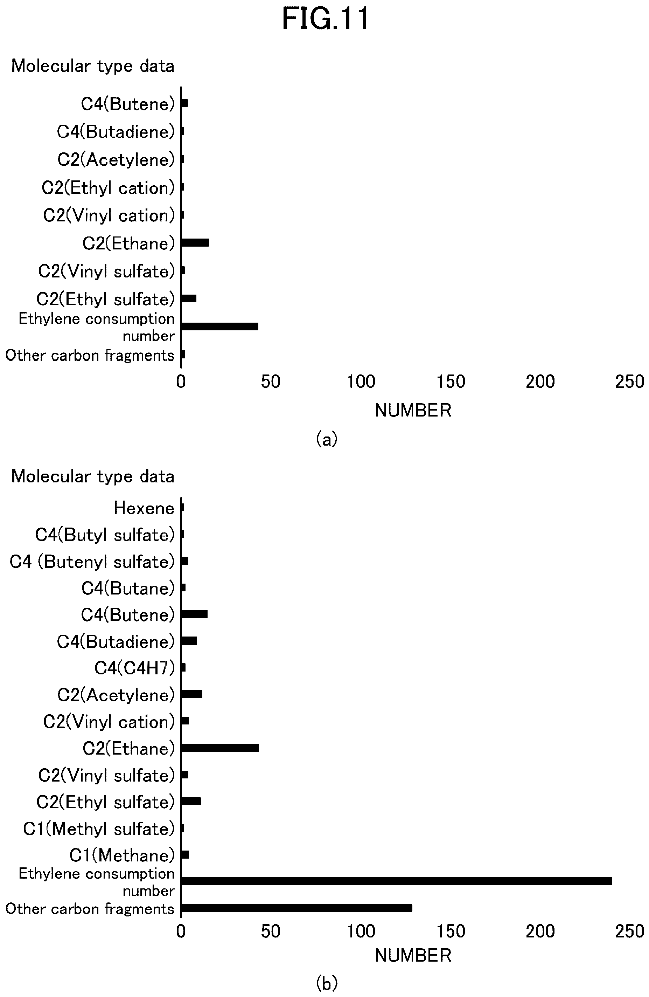

Next, specific examples of the processed result of the molecular dynamics computation process and the elementary reaction data will be described. FIG. 11 is a diagram illustrating examples of the processed results of the molecular dynamics computation process.

FIG. 11 (a) and (b) are examples of the molecular type data and the number, which are the processed results of the molecular dynamics computation process for a case where:
- A reaction system composed of 1 to 20000 molecules is generated, and (ethylene molecule, 432) and (sulfuric acid, 216) are input as the initial molecule information (type, number);
- (butene, 3) is input as the target molecule information (type, number);
- (450K, cube with a side of 5 nm) is input as the reaction conditions (cell temperature, cell volume);
- (0.5 ns, ON) is input as the initial conditions (width of time step, temperature adjusting algorithm) of molecular dynamics computation; and
- (molecule-to-molecule of close proximity molecules including sulfur molecules, 2 ps, 40 kcal/mol (FIG. 11 (a)) or 160 kcal/mol (FIG. 11 (b))) are input as the reaction sampling method (target to which the translational velocity is not to be imparted, time interval at which the translational velocity is to be imparted, the translational velocity to be imparted).

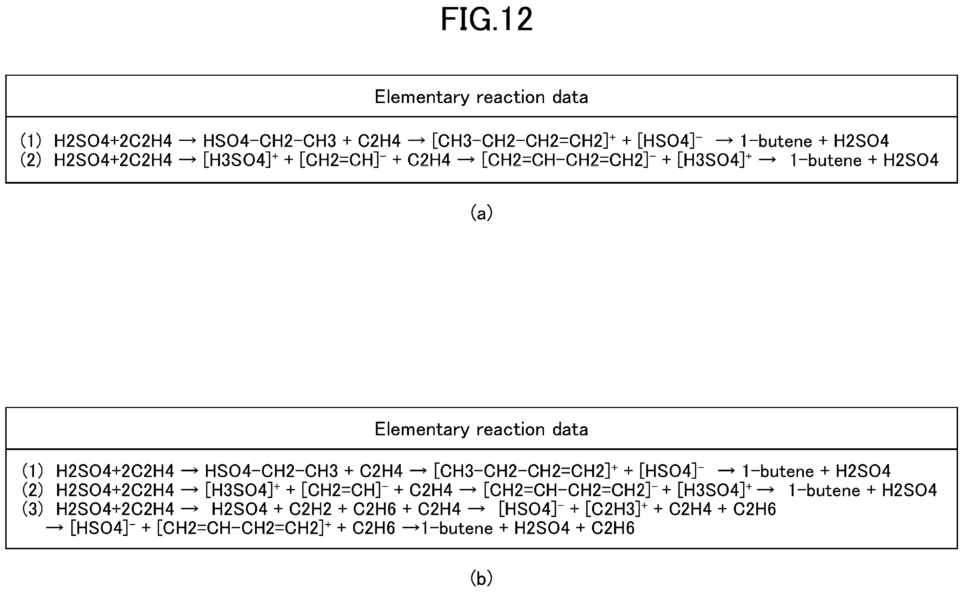

In addition, the examples illustrated in FIG. 12 (a) and (b) are the elementary reaction data corresponding to a case where the translational velocity is 40 kcal/mol and a case where the translational velocity is 160 kcal/mol, respectively, and indicate that the following elementary reaction was confirmed in both the case where the translational velocity is 40 kcal/mol and the case where the translational velocity is 160 kcal/mol:
- H2SO4+2C2H4→4HS04-CH2-CH3+C2H4→[CH3-CH2-CH2=CH2]⁺+[HSO4]⁻→1-butene+H2SO4; and
- H2SO4+2C2H4→[H3SO4]⁺+[CH2=CH]⁻+C2H4.

Moreover, the example illustrated in FIG. 12 (b) illustrates that the following elementary reaction was confirmed only in the case where the translational velocity is 160 kcal/mol:
- H2SO4+2C2H4→H2SO4+C2H2+C2H6+C2H4→[HSO4]⁻ +[C2H3]⁺+C2H4+C2H6→[HSO4]⁻+[CH2+CH-CH2=CH2]⁺+C2H6→1-butene+H2SO4+C2H6.

Accordingly, by varying the reaction sampling method, the elementary reaction data varies as a result of the molecular dynamics computation process, and thus, the user can perform a more useful analysis when analyzing the reaction mechanism by appropriately inputting the reaction sampling method.

### <Conclusion>

As is clear from the description above, the simulation device 100 according to the first embodiment includes:
- While performing the molecular dynamics computation, the distance between the atom forming the first molecule and the atom forming the second molecule is computed, to determine whether the first molecule and the second molecule are in close proximity of each other;
- When the first molecule and the second molecule are determined to be in close proximity of each other, the parameter for increasing the reaction probability is imparted to the first molecule and the second molecule;
- The elementary reaction in the transition state is generated based on the chemical reaction identified in the molecular dynamics computation; and
- The reaction rate constant of the generated elementary reaction is computed.

Hence, according to the first embodiment, even in a case where the temperature of the cell is lowered as the reaction condition, it is possible to promote the reaction that can actually occur. That is, according to the first embodiment, the reaction that cannot actually occur or the reaction that hardly occurs can be made less likely to be output as the simulation result, and the reaction that can actually occur can be made more likely to be output as the simulation result. As a result, according to the first embodiment, it is possible to perform a more useful analysis when analyzing the reaction mechanism by the molecular dynamics computation.

### [Second Embodiment]

In the first embodiment, a method of setting the atomic mass number (the mass of each individual atom per 1 mol) when performing the molecular dynamics computation process is not described. However, the atomic mass number may be set to an actual mass number or a mass number different from the actual mass number. For example, the mass number of all of the atoms may be set to the same mass number. In general, in the molecular dynamics computation, the width (Δt) of the time step needs to be set small when an atom having a small mass number is included. This is because, in the case of the atom having a small mass number, if the width (Δt) of the time step is too large, the distance moved for each width (Δt) of the time step will become long.

In contrast, in the case where the mass number of all of the atoms is set to the same mass number, the width (Δt) of the time step can be set large, and a computational complexity of the computation performed by the simulation device 100 can be reduced. Because the number of reacting atoms does not change even when the mass number of the atom to be set is changed, the analysis of the reaction mechanism is unaffected thereby.

In addition, in the first embodiment described above, the "target to which the translational velocity is not to be imparted" is input as the reaction sampling method. However, the "target to which the translational velocity is to be imparted" may be input as the reaction sampling method, in place of the "target to which the translational velocity is not to be imparted" or in addition to the "target to which the translational velocity is not to be imparted". In this example, in a case where the molecule identified as the close proximity molecule is a molecule that does not correspond to the target to which the translational velocity is to be imparted, the molecular dynamics computation unit 310 does not impart the translational velocity to the molecule identified as the close proximity molecule.

Moreover, in the above description, the type of atom is specified when inputting the "target to which the translational velocity is to be imparted" or the "target to which the translational velocity is not to be imparted" as the reaction sampling method. However, when inputting the "target to which translational velocity is to be imparted" or the "target to which translational velocity is not to be imparted", an index of an atom may be specified instead of specifying the type of atom, for example.

Further, in the first embodiment described above, the "translational velocity to be imparted" is input as the reaction sampling method. However, a kinetic energy itself to be imparted may be input instead of inputting the translational velocity. Alternatively, a configuration may be adopted to input information related to a temperature for increasing the kinetic energy of the molecule. In this case, the imparting unit 314 imparts the information related to the kinetic energy or the temperature to the close proximity molecule, in place of the translational velocity.

In addition, although the first embodiment described above is configured to impart the translational velocity to both of the close proximity molecules, a configuration may be adopted to impart the translational velocity to one of the close proximity molecules.

Usage scenarios of the simulation device 100 according to the first embodiment are not described above, however, the analysis results of the reaction mechanism analyzed by the simulation device 100 may be reflected to manufacturing conditions of manufacturing processes of the product, for example. More particularly, by performing the simulation process while varying the information (the initial molecule information, the target molecule information, the reaction conditions, the initial conditions of the molecular dynamics computation, and the reaction sampling method) input to the setting unit 311, the reaction mechanism can be analyzed to derive the manufacturing conditions of the manufacturing processes. Then, the manufacturing processes of the product may be performed under the derived manufacturing conditions. Alternatively, the reaction mechanism can be analyzed by to derive an optimum manufacturing method of the product, by performing the simulation process while varying the information (the initial molecule information, the target molecule information, the reaction conditions, the initial conditions of the molecular dynamics computation, and the reaction sampling method) input to the setting unit 311. Further, the manufacturing processes of the product may be generated under the derived manufacturing method.

This application is based upon and claims priority to Japanese Patent Application No. 2021-142374, filed on September 1, 2021.

### DESCRIPTION OF THE REFERENCE NUMERALS

- 100:: Simulation device
- 310:: Molecular dynamics computation unit
- 311:: Setting unit
- 312:: Position computation unit
- 313:: Determination unit
- 314:: Imparting unit
- 320:: Elementary reaction generation unit
- 330:: Optimization unit
- 340:: Reaction rate constant computation unit

## Claims

1. A simulation device comprising:
a determination unit (313) configured to compute a distance between an atom forming a first molecule and an atom forming a second molecule while performing a molecular dynamics computation, and determine whether or not the first molecule and the second molecule are in close proximity of each other;
an imparting unit (314) configured to impart a parameter for increasing a reaction probability with respect to the first molecule and/or the second molecule in a case where the determination unit determines that the first molecule and the second molecule are in close proximity of each other;
an elementary reaction generation unit (320) configured to generate an elementary reaction in a transition state, based on a chemical reaction identified in the molecular dynamics computation; and
a reaction rate constant computation unit (340) configured to compute a reaction rate constant of the generated elementary reaction.

2. The simulation device as claimed in claim 1, further comprising:
a setting unit (311) configured to set a time interval at which the imparting unit (314) is configured to impart the parameter before performing the molecular dynamics computation,
wherein the imparting unit (314) is configured to impart the parameter in a case where the determination unit determines the close proximity and the time interval elapses from a previous imparting.

3. The simulation device as claimed in claim 2, wherein the setting unit (311) is configured to set a time interval that does not affect a temperature of an entire reaction system, and is required for a molecule after the chemical reaction to stabilize.

4. The simulation device as claimed in claim 1, further comprising:
a setting unit (311) configured to set a target to which the parameter is not to be imparted by the imparting unit before performing the molecular dynamics computation,
wherein the imparting unit (314) is configured not to impart the parameter with respect to each of the first molecule and the second molecule even in a case where the determination unit (313) is configured to determine the proximity when both of the first molecule and the second molecule are targets to which the parameter is not to be imparted.

5. The simulation device as claimed in claim 4, wherein
the setting unit sets (311) is configured to set a target to which the parameter is not to be imparted by the imparting unit (314), by specifying a type of atom or an index of atom, and
the imparting unit (314) is configured not to impart the parameter with respect to each of the first molecule and the second molecule even in a case where the determination unit (313) is configured to determine that the close proximity when both of the first molecule and the second molecule are molecules including the specified atom.

6. The simulation device as claimed in claim 1, further comprising:
a position computation unit (312) configured to identify atom-to-atom existing within a range of a bond distance as the first molecule or the second molecule.

7. The simulation device as claimed in claim 6, wherein the determination unit (313) is configured to determine that the first molecule and the second molecule are in close proximity of each other in a case where the distance between the atom included in the first molecule and the atom included in the second molecule is a predetermined multiple or less of the bond distance.

8. The simulation device as claimed in claim 1, further comprising:
a setting unit (311) configured to set the parameter to be imparted by the imparting unit before performing the molecular dynamics computation,
wherein the imparting unit (314) is configured to impart information related to a temperature, based on the parameter set by the setting unit (311).

9. The simulation device as claimed in claim 8, wherein the imparting unit (314) is configured to impart a kinetic energy in a colliding direction, based on the parameter set by the setting unit.

10. The simulation device as claimed in claim 8, wherein the setting unit (311) is configured to set the information related to the temperature to be imparted as the parameter.

11. The simulation device as claimed in claim 9, wherein the setting unit (311) is configured to set the kinetic energy to be imparted as the parameter.

12. The simulation device as claimed in claim 9, wherein the setting unit (311) is configured to set a translational velocity to be imparted as the parameter.

13. A simulation method comprising the steps of:
determining whether or not a first molecule and a second molecule are in close proximity of each other, by computing a distance between an atom forming the first molecule and an atom forming the second molecule while performing a molecular dynamics computation;
imparting a parameter for increasing a reaction probability with respect to the first molecule and/or the second molecule in a case where the determining step determines that the first molecule and the second molecule are in close proximity of each other;
generating an elementary reaction in a transition state, based on a chemical reaction identified in the molecular dynamics computation; and
computing a reaction rate constant of the generated elementary reaction.

14. A simulation program for causing a computer to perform the steps of:
determining whether or not a first molecule and a second molecule are in close proximity of each other, by computing a distance between an atom forming the first molecule and an atom forming the second molecule while performing a molecular dynamics computation;
imparting a parameter for increasing a reaction probability with respect to the first molecule and/or the second molecule in a case where the determining step determines that the first molecule and the second molecule are in close proximity of each other;
generating an elementary reaction in a transition state, based on a chemical reaction identified in the molecular dynamics computation; and
computing a reaction rate constant of the generated elementary reaction.

## Patentansprüche

1. Simulationsvorrichtung, umfassend:
eine Bestimmungseinheit (313), die konfiguriert ist, einen Abstand zwischen einem Atom, das ein erstes Molekül bildet, und einem Atom, das ein zweites Molekül bildet, zu berechnen, während eine Molekulardynamik-Berechnung durchgeführt wird, und zu bestimmen, ob das erste Molekül und das zweite Molekül in enger Nähe zueinander sind oder nicht;
eine Übertragungseinheit (314), die konfiguriert ist, einen Parameter zur Erhöhung einer Reaktionswahrscheinlichkeit in Bezug auf das erste Molekül und/oder das zweite Molekül in einem Fall zu übertragen, in dem die Bestimmungseinheit bestimmt, dass das erste Molekül und das zweite Molekül in enger Nähe zueinander sind;
eine Elementarreaktions-Erzeugungseinheit (320), die konfiguriert ist, eine Elementarreaktion in einem Übergangszustand zu erzeugen, basierend auf einer chemischen Reaktion, die in der Molekulardynamik-Berechnung identifiziert wurde; und
eine Reaktionsratenkonstanten-Berechnungseinheit (340), die konfiguriert ist, eine Reaktionsratenkonstante der erzeugten Elementarreaktion zu berechnen.

2. Simulationsvorrichtung nach Anspruch 1, ferner umfassend:
eine Einstelleinheit (311), die konfiguriert ist, ein Zeitintervall einzustellen, in dem die Übertragungseinheit (314) konfiguriert ist, den Parameter zu übertragen, bevor die Molekulardynamik-Berechnung durchgeführt wird,
wobei die Übertragungseinheit (314) konfiguriert ist, den Parameter in einem Fall zu übertragen, in dem die Bestimmungseinheit die enge Nähe bestimmt und das Zeitintervall von einer vorherigen Übertragung verstreicht.

3. Simulationsvorrichtung nach Anspruch 2,
wobei die Einstelleinheit (311) konfiguriert ist, ein Zeitintervall einzustellen, das eine Temperatur eines gesamten Reaktionssystems nicht beeinflusst und erforderlich ist, damit sich ein Molekül nach der chemischen Reaktion stabilisiert.

4. Simulationsvorrichtung nach Anspruch 1, ferner umfassend:
eine Einstelleinheit (311), die konfiguriert ist, ein Ziel einzustellen, auf das der Parameter von der Übertragungseinheit nicht übertragen werden soll, bevor die Molekulardynamik-Berechnung durchgeführt wird,
wobei die Übertragungseinheit (314) konfiguriert ist, den Parameter nicht in Bezug auf jedes des ersten Moleküls und des zweiten Moleküls auch in einem Fall zu übertragen, in dem die Bestimmungseinheit (313) konfiguriert ist, die Nähe zu bestimmen, wenn sowohl das erste Molekül als auch das zweite Molekül Ziele sind, auf die der Parameter nicht übertragen werden soll.

5. Simulationsvorrichtung nach Anspruch 4, wobei
die Einstelleinheit (311) konfiguriert ist, ein Ziel einzustellen, auf das der Parameter von der Übertragungseinheit (314) nicht übertragen werden soll, durch Spezifizierung eines Atomtyps oder eines Atomindex, und
die Übertragungseinheit (314) konfiguriert ist, den Parameter nicht in Bezug auf jedes des ersten Moleküls und des zweiten Moleküls auch in einem Fall zu übertragen, in dem die Bestimmungseinheit (313) konfiguriert ist zu bestimmen, dass die enge Nähe vorliegt, wenn sowohl das erste Molekül als auch das zweite Molekül Moleküle sind, die das spezifizierte Atom enthalten.

6. Simulationsvorrichtung nach Anspruch 1, ferner umfassend:
eine Positionsberechnungseinheit (312), die konfiguriert ist, Atom-zu-Atom innerhalb eines Bereichs eines Bindungsabstands als das erste Molekül oder das zweite Molekül zu identifizieren.

7. Simulationsvorrichtung nach Anspruch 6, wobei die Bestimmungseinheit (313) konfiguriert ist zu bestimmen, dass das erste Molekül und das zweite Molekül in enger Nähe zueinander sind, in einem Fall, in dem der Abstand zwischen dem im ersten Molekül enthaltenen Atom und dem im zweiten Molekül enthaltenen Atom ein vorbestimmtes Vielfaches oder weniger des Bindungsabstands ist.

8. Simulationsvorrichtung nach Anspruch 1, ferner umfassend:
eine Einstelleinheit (311), die konfiguriert ist, den von der Übertragungseinheit zu übertragenden Parameter einzustellen, bevor die Molekulardynamik-Berechnung durchgeführt wird,
wobei die Übertragungseinheit (314) konfiguriert ist, basierend auf dem von der Einstelleinheit (311) eingestellten Parameter Informationen bezüglich einer Temperatur zu übertragen.

9. Simulationsvorrichtung nach Anspruch 8,
wobei die Übertragungseinheit (314) konfiguriert ist, basierend auf dem von der Einstelleinheit eingestellten Parameter eine kinetische Energie in einer Kollisionsrichtung zu übertragen.

10. Simulationsvorrichtung nach Anspruch 8,
wobei die Einstelleinheit (311) konfiguriert ist, die temperaturrelevanten Informationen, die als Parameter übertragen werden sollen, einzustellen.

11. Simulationsvorrichtung nach Anspruch 9,
wobei die Einstelleinheit (311) konfiguriert ist, die als Parameter zu übertragende kinetische Energie einzustellen.

12. Simulationsvorrichtung nach Anspruch 9,
wobei die Einstelleinheit (311) konfiguriert ist, eine als Parameter zu übertragende Translationsgeschwindigkeit einzustellen.

13. Simulationsverfahren, umfassend die Schritte:
Bestimmen, ob ein erstes Molekül und ein zweites Molekül in enger Nähe zueinander sind oder nicht, durch Berechnung eines Abstands zwischen einem Atom, das das erste Molekül bildet, und einem Atom, das das zweite Molekül bildet, während eine Molekulardynamik-Berechnung durchgeführt wird;
Übertragen eines Parameters zur Erhöhung einer Reaktionswahrscheinlichkeit in Bezug auf das erste Molekül und/oder das zweite Molekül in einem Fall, in dem der Bestimmungsschritt bestimmt, dass das erste Molekül und das zweite Molekül in enger Nähe zueinander sind;
Erzeugen einer Elementarreaktion in einem Übergangszustand, basierend auf einer chemischen Reaktion, die in der Molekulardynamik-Berechnung identifiziert wurde; und
Berechnen einer Reaktionsratenkonstante der erzeugten Elementarreaktion.

14. Simulationsprogramm zum Veranlassen eines Computers, die Schritte
durchzuführen:
Bestimmen, ob ein erstes Molekül und ein zweites Molekül in enger Nähe zueinander sind oder nicht, durch Berechnung eines Abstands zwischen einem Atom, das das erste Molekül bildet, und einem Atom, das das zweite Molekül bildet, während eine Molekulardynamik-Berechnung durchgeführt wird;
Übertragen eines Parameters zur Erhöhung einer Reaktionswahrscheinlichkeit in Bezug auf das erste Molekül und/oder das zweite Molekül in einem Fall, in dem der Bestimmungsschritt bestimmt, dass das erste Molekül und das zweite Molekül in enger Nähe zueinander sind;
Erzeugen einer Elementarreaktion in einem Übergangszustand, basierend auf einer chemischen Reaktion, die in der Molekulardynamik-Berechnung identifiziert wurde; und
Berechnen einer Reaktionsratenkonstante der erzeugten Elementarreaktion.

## Revendications

1. Dispositif de simulation comprenant :
une unité de détermination (313) configurée pour calculer une distance entre un atome d'une première molécule et un atome d'une deuxième molécule lors de l'exécution d'un calcul de dynamique moléculaire, et pour déterminer si la première molécule et la deuxième molécule sont ou non à proximité l'une de l'autre ;
une unité d'attribution (314) configurée pour attribuer un paramètre permettant d'augmenter une probabilité de réaction par rapport à la première molécule et/ou à la deuxième molécule dans le cas où l'unité de détermination détermine que la première molécule et la deuxième molécule sont très proches l'une de l'autre ;
une unité de génération de réaction élémentaire (320) configurée pour générer une réaction élémentaire dans un état de transition, basée sur une réaction chimique identifiée dans le calcul de dynamique moléculaire ; et
une unité de calcul de constante de vitesse de réaction (340) configurée pour calculer la constante de vitesse de réaction de la réaction élémentaire générée.

2. Dispositif de simulation selon la revendication 1, comprenant en outre :
une unité de réglage (311) configurée pour définir un intervalle de temps pendant lequel l'unité d'attribution (314) est configurée pour attribuer le paramètre avant d'effectuer le calcul de dynamique moléculaire, dans lequel l'unité d'attribution (314) est configurée pour attribuer le paramètre dans le cas où l'unité de détermination détermine la proximité et l'intervalle de temps écoulé depuis une précédente attribution.

3. Dispositif de simulation selon la revendication 2, dans lequel l'unité de réglage (311) est configurée pour définir un intervalle de temps qui n'affecte pas la température de l'ensemble du système de réaction et qui est nécessaire à la stabilisation d'une molécule après la réaction chimique.

4. Dispositif de simulation selon la revendication 1, comprenant en outre :
une unité de réglage (311) configurée pour définir une cible à laquelle le paramètre ne doit pas être attribué par l'unité d'attribution avant d'effectuer le calcul de dynamique moléculaire,
dans lequel l'unité d'attribution (314) est configurée pour ne pas attribuer le paramètre à chacune des première et deuxième molécules, même dans le cas où l'unité de détermination (313) est configurée pour déterminer la proximité lorsque les deux molécules, la première et la deuxième, sont des cibles auxquelles le paramètre ne doit pas être attribué.

5. Dispositif de simulation selon la revendication 4, dans lequel
L'unité de réglage (311) est configurée pour définir une cible à laquelle le paramètre ne doit pas être attribué par l'unité d'attribution (314), en spécifiant un type d'atome ou un indice d'atome, et
l'unité d'attribution (314) est configurée pour ne pas attribuer le paramètre par rapport à chacune des première et deuxième molécules, même dans le cas où l'unité de détermination (313) est configurée pour déterminer que la proximité est grande lorsque les deux molécules, la première et la deuxième, contiennent l'atome spécifié.

6. Dispositif de simulation selon la revendication 1, comprenant en outre : une unité de calcul de position (312) configurée pour identifier une liaison atome-à-atome existant dans une plage de distance de liaison comme étant la première molécule ou la deuxième molécule.

7. Dispositif de simulation selon la revendication 6, dans lequel l'unité de détermination (313) est configurée pour déterminer que la première molécule et la deuxième molécule sont proches l'une de l'autre dans le cas où la distance entre l'atome inclus dans la première molécule et l'atome inclus dans la deuxième molécule est un multiple prédéterminé ou inférieur à la distance de liaison.

8. Dispositif de simulation selon la revendication 1, comprenant en outre :
une unité de réglage (311) configurée pour définir le paramètre à attribuer par l'unité d'attribution avant d'effectuer le calcul de dynamique moléculaire,
dans lequel l'unité d'attribution (314) est configurée pour attribuer des informations relatives à une température, en fonction du paramètre défini par l'unité de réglage (311).

9. Dispositif de simulation selon la revendication 8, dans lequel l'unité d'attribution (314) est configurée pour attribuer une énergie cinétique dans une direction de collision, en fonction du paramètre défini par l'unité de réglage.

10. Dispositif de simulation selon la revendication 8, dans lequel l'unité de réglage (311) est configurée pour définir les informations relatives à la température à attribuer en tant que paramètre.

11. Dispositif de simulation selon la revendication 9, dans lequel l'unité de réglage (311) est configurée pour définir l'énergie cinétique à attribuer en tant que paramètre.

12. Dispositif de simulation selon la revendication 9, dans lequel l'unité de réglage (311) est configurée pour définir une vitesse de translation à attribuer en tant que paramètre.

13. Procédé de simulation comprenant les étapes suivantes :
déterminer si une première molécule et une deuxième molécule sont proches l'une de l'autre, en calculant une distance entre un atome de la première molécule et un atome de la deuxième molécule tout en effectuant un calcul de dynamique moléculaire ;
attribuer un paramètre pour augmenter la probabilité d'une réaction par rapport à la première molécule et/ou à la deuxième molécule dans un cas où l'étape de détermination établit que la première molécule et la deuxième molécule sont très proches l'une de l'autre ;
générer une réaction élémentaire dans un état de transition, basée sur une réaction chimique identifiée dans le calcul de dynamique moléculaire ; et
calculer la constante de vitesse de réaction de la réaction élémentaire générée.

14. Programme de simulation permettant à un ordinateur d'exécuter les étapes suivantes :
déterminer si une première molécule et une deuxième molécule sont proches l'une de l'autre, en calculant une distance entre un atome de la première molécule et un atome de la deuxième molécule tout en effectuant un calcul de dynamique moléculaire ;
attribuer un paramètre pour augmenter la probabilité d'une réaction par rapport à la première molécule et/ou à la deuxième molécule dans un cas où l'étape de détermination établit que la première molécule et la deuxième molécule sont très proches l'une de l'autre ;
générer une réaction élémentaire dans un état de transition, basée sur une réaction chimique identifiée dans le calcul de dynamique moléculaire ; et
calculer la constante de vitesse de réaction de la réaction élémentaire générée.
